# EUROPÄISCHE PATENTSCHRIFT

(11) **EP 1 783 230 B1**
(45) Veröffentlichungstag und Bekanntmachung des Hinweises auf die Patenterteilung: **10.12.2008**
(21) Anmeldenummer: 07102437.6
(22) Anmeldetag: 02.10.2003
(51) Int. Cl.: C12P 13/12, C12N 9/10, C12N 15/09

(54) **Feedback-resistente Homoserin-Transsuccinylasen**
Feedback-resistent homoserin-transsuccinylases
Homosérine transsuccinylases resistantes à la rétroaction

(30) Priorität: 11.10.2002 DE 10247437
(43) Veröffentlichungstag der Anmeldung: 09.05.2007
(62) Teilanmeldung aus: 03767502.2
(73) Patentinhaber: Wacker Chemie AG, 81737 München (DE)
(72) Erfinder: Leonhartsberger, Susanne, 07743, Jena (DE); Pfeiffer-Schwiesow, Kerstin, 81369, München (DE); Bauer, Brigitte, 80798, München (DE); Winterhalter, Christoph, 82343, Pöcking (DE)
(74) Vertreter: Potten, Holger

(56) Entgegenhaltungen:
- US-A- 5 698 418
- US-A1- 2002 106 800
- DATABASE WPI Section Ch, Week 200103 Derwent Publications Ltd., London, GB; Class B05, AN 2001-018703 XP002273153 & JP 2000 139471 A (AJINOMOTO KK) 23. Mai 2000 (2000-05-23)
- DUCLOS B ET AL: "NUCLEOTIDE SEQUENCE OF THE MET-A GENE ENCODING HOMOSERINE TRANS SUCCINYLASE IN ESCHERICHIA-COLI" NUCLEIC ACIDS RESEARCH, Bd. 17, Nr. 7, 1989, Seite 2856, XP002273152 & ISSN: 0305-1048
- CHATTOPADHYAY M K ET AL: "CONTROL OF METHIONINE BIOSYNTHESIS IN ESCHERICHIA-COLI K12 A CLOSER STUDY WITH ANALOGUE-RESISTANT MUTANTS" JOURNAL OF GENERAL MICROBIOLOGY, SOCIETY FOR MICROBIOLOGY, READING, GB, Bd. 137, Nr. 3, 1991, Seiten 685-691, XP009041956 ISSN: 0022-1287

### SEQUENCE LISTING

<110> Consortium fuer elektrochemische Industrie GmbH
<120> Feedback-resistente Homoserin-Transsuccinylasen
<130> CO10217
<140>
   <141>
<160> 24
<170> PatentIn Ver. 2.0
<210> 1
   <211> 930
   <212> DNA
   <213> Escherichia coli
<220>
   <221> CDS
   <222> (1)..(930)
<300>
   <301> Blattner, F. R.
   <302> The complete genome sequence of Escherichia coli K-12.
   <303> Science
   <304> 277
   <305> 5331
   <306> 1453-1474
<307> 1997
<400> 1
   atg ccg att cgt gtg ccg gac gag cta ccc gcc gtc aat ttc ttg cgt    48
   gaa gaa aac gtc ttt gtg atg aca act tct cgt gcg tct ggt cag gaa    96
   att cgt cca ctt aag gtt ctg atc ctt aac ctg atg ccg aag aag att   144
   gaa act gaa aat cag ttt ctg cgc ctg ctt tca aac tca cct ttg cag   192
   gtc gat att cag ctg ttg cgc atc gat tcc cgt gaa tcg cgc aac acg   240
   ccc gca gag cat ctg aac aac ttc tac tgt aac ttt gaa gat att cag   288
   gat cag aac ttt gac ggt ttg att gta act ggt gcg ccg ctg ggc ctg   336
   gtg gag ttt aat gat gtc gct tac tgg ccg cag atc aaa cag gtg ctg      384
   gag tgg tcg aaa gat cac gtc acc tcg acg ctg ttt gtc tgc tgg gcg      432
   gta cag gcc gcg ctc aat atc ctc tac ggc att cct aag caa act cgc      480
   acc gaa aaa ctc tct ggc gtt tac gag cat cat att ctc cat cct cat      528
   gcg ctt ctg acg cgt ggc ttt gat gat tca ttc ctg gca ccg cat tcg      576
   cgc tat gct gac ttt ccg gca gcg ttg att cgt gat tac acc gat ctg      624
   gaa att ctg gca gag acg gaa gaa ggg gat gca tat ctg ttt gcc agt      672
   aaa gat aag cgc att gcc ttt gtg acg ggc cat ccc gaa tat gat gcg    720
   caa acg ctg gcg cag gaa ttt ttc cgc gat gtg gaa gcc gga cta gac    768
   ccg gat gta ccg tat aac tat ttc ccg cac aat gat ccg caa aat aca    816
   ccg cga gcg agc tgg cgt agt cac ggt aat tta ctg ttt acc aac tgg    864
   ctc aac tat tac gtc tac cag atc acg cca tac gat cta cgg cac atg    912
   aat cca acg ctg gat taa                                            930
<210> 2
   <211> 309
   <212> PRT
   <213> Escherichia coli
<400> 2
<210> 3
   <211> 30
   <212> DNA
   <213> Artificial Sequence
<220>
   <223> Description of Artificial Sequence: Oligonukleotid metAfw
<400> 3
   gatcccatgg ctccttttag tcattcttat 30
<210> 4
   <211> 36
   <212> DNA
   <213> Artificial Sequence
<220>
   <223> Description of Artificial Sequence: Oligonukleotid metArev
<400> 4
   gatcgagctc agtactatta atccagcgtt ggattc 36
<210> 5
   <211> 33
   <212> DNA
   <213> Artificial Sequence
<220>
   <223> Description of Artificial Sequence: Oligonukleotid GAPDHfw
<400> 5
   gtcgacgcgt gaggcgagtc agtcgcgtaa tgc 33
<210> 6
   <211> 42
   <212> DNA
   <213> Artificial Sequence
<220>
   <223> Description of Artificial Sequence: Oligonukleotid GAPDHrevII
<400> 6
   gaccttaatt aagatctcat atgttccacc agctatttgt ta 42
<210> 7
   <211> 37
   <212> DNA
   <213> Artificial Sequence
<220>
   <223> Description of Artificial Sequence: Oligonukleotid metAfw2
<400> 7
   catggctcct tttagtcatt cttatattct aacgtag 37
<210> 8
   <211> 47
   <212> DNA
   <213> Artificial Sequence
<220>
   <223> Description of Artificial Sequence: Oigonukleotid metArev2
<400> 8
   acgcgtatgc atccagagct cagtactatt aatccagcgt tggattc 47
<210> 9
   <211> 25
   <212> DNA
   <213> Artificial Sequence
<220>
   <223> Description of Artificial Sequence: Oligonukleotid metAmutfw1
<400> 9
   nnncagatca cgccatacga tctac 25
<210> 10
   <211> 23
   <212> DNA
   <213> Artificial Sequence
<220>
   <223> Description of Artificial Sequence: Oligonukleotid metAmutrev1
<400> 10
   gacgtaatag ttgagccagt tgg 23

## Beschreibung

Die vorliegende Erfindung betrifft feedback-resistente Homoserin-Transsuccinylasen, Mikroorganismenstämme enthaltend diese Enzyme sowie ihre Verwendung zur Herstellung von L-Methionin oder S-Adenosylmethionin.

Methionin ist eine für den Menschen und für viele Tiere essentielle Aminosäure. Sie wird vor allem für den Futtermittelmarkt produziert und als Racemat dem Tierfutter zugesetzt. Die Synthese erfolgt chemisch aus Acrolein und Methanthiol über 3-(Methylthio)-propionaldehyd, der mit Blausäure, Ammoniak und Kohlendioxid über ein Hydantoin in D,L-Methionin überführt wird. Eine Racemattrennung kann enzymatisch erfolgen.

S-Adenosylmethionin (SAM) ist der wichtigste Methylgruppendonor im Stoffwechsel und findet im Pharmabereich Verwendung bei der Behandlung von Depressionen, Erkrankungen der Leber und Arthritis. Beschriebene Verfahren zur SAM-Herstellung umfassen vor allem die Anzucht von Hefen (Schlenk F. und DePalma R.E., J. Biol. Chem. 1037-1050 (1957), Shiozaki S. et al., Agric. Biol. Chem. 53, 3269-3274 (1989)) in Gegenwart der Vorstufe L-Methionin und die chromatographische Aufreinigung nach Autolyse.

Die mikrobielle Synthese von Methionin wurde besonders intensiv im Bakterium E. coli untersucht (Greene, R.C., Biosynthesis of Methionine in: Neidhardt F.C., Escherichia coli and Salmonella typhimurium, Cellular and molecular biology, Second Edition, ASM Press, Washington DC (1996), Seiten 542-560 und darin enthaltenen Referenzen). Sie besteht aus einer Reihe von durch Enzyme katalysierten Reaktionen und ist streng reguliert. Die ersten Schritte der Synthese ausgehend von Aspartat bis zu Homoserin verlaufen für die Bildung der Aminosäuren Threonin, Leucin, Isoleucin und Valin parallel. Der erste für die Methioninsynthese spezifische Schritt ist die Bildung von O-Succinyl-Homoserin aus Succinyl-CoA und Homoserin unter Abspaltung von Coenzym A. Diese Reaktion wird durch das Enzym Homoserin-Succinyltransferase (Homoserin-O-Transsuccinylase, MetA, EC 2.3.1.46) katalysiert. Die Synthese von SAM erfolgt in einem Schritt aus L-Methionin und ATP.

Die Aktivität der Homoserin-Transsuccinylase ist in Gegenwart von L-Methionin und/oder SAM gehemmt (Lee L.-W. et al., J. Biol. Chem. 241, 5479-5480 (1966)). Diese Endprodukthemmung verhindert einerseits im Bakterium eine überschüssige, energieverbrauchende Synthese von Methionin und SAM, steht andererseits jedoch auch einer mikrobiellen Produktion dieser beiden Substanzen im industriellen Maßstab im Weg. Das für die Homoserin-Transsuccinylase codierende Gen besteht aus 930 (inklusive Stopcodon) Basenpaaren, das davon codierte Protein aus 309 Aminosäuren. Bisher wurde die Struktur der Homoserin-Transsuccinylase nicht aufgeklärt und daher ist auch eine Identifizierung der an einer Endprodukthemmung beteiligten Aminosäuren nicht möglich.

Eine bekannte Methode, die Synthese von Stoffwechselendprodukten zu verstärken ist die Verwendung von veränderten Enzymen, deren Aktivität nicht mehr hemmbar durch das Endprodukt ihres Stoffwechselweges ist (feedback-resistente Mutanten). So wurden beispielsweise feedback-resistente Mutanten der 3-Desoxy-D-Arabinoheptulonsäure-7-Phosphat-Synthase für die Steigerung der Synthese von L-Tryptophan und L-Phenylalanin hergestellt (EP0745671A2) und feedback-resistente Mutanten der Chorismat-Mutase/Prephenat-Dehydratase zur Steigerung der Phenylalanin-Produktion erzeugt (US5120837).

Vor kurzem wurde das Enzym Homoserin-Transsuccinylase aus E. coli durch Mutation der dafür codierenden DNS-Sequenz dahingehend verändert, dass die entstandenen Proteine eine deutlich verringerte Hemmbarkeit ihrer Aktivität in Gegenwart von 1 mM L-Methionin oder 1 mM SAM aufweisen (JP2000139471A). Es handelt sich dabei um folgende Aminosäureaustausche: Arginin an Position 27 wurde durch Cystein ersetzt, Isoleucin an Position 296 durch Serin und Prolin an Position 298 durch Leucin. Die veränderten Homoserin-Transsuccinylasen zeigten in Gegenwart von 1 mM L-Methionin Restaktivitäten zwischen 44 und 89%, in Gegenwart von 1 mM SAM zwischen 10 und 73%. Bakterienstämme, die diese veränderten Proteine enthalten, zeigen gesteigerte L-Methionin-Produktion. Jedoch weisen diese veränderten Homoserin-Transsuccinylasen in Abwesenheit von L-Methionin und SAM eine im Vergleich zum Wildtyp deutlich verringerte Aktivität auf. Aus Database WPI Section Ch, Week 200103 Derwent Publications Ltd., London, GB; Class B05, AN 2001-018703 XP002273153 & JP 2000 139471 A (AJINOMOTO KK) 23. Mai 2000 (2000-05-23) ist eine DNS bekannt, welche die folgenden Mutationen in der 309 Aminosäuren umfassenden Sequenz aufweist: Arg27 -> Cys; lle296 -> Ser; Pro298 -> Leu; Arg27 -> Cys und lle296 -> Ser; lle296 -> Ser und Pro298 -> Leu; Pro298 -> Leu und Arg27 -> Cys; oder Arg27 -> Cys, lle296 -> Ser und Pro298 -> Leu. Es ist wünschenswert, möglichst viele Varianten der Homoserin-Transsuccinylase, die sich im Grad ihrer Aktivität und im Grad ihrer Hemmbarkeit durch L-Methionin und/oder SAM unterscheiden, zur Verfügung zu haben, da die mikrobielle Biosynthese von L-Methionin und SAM in ihrem Ablauf und ihrer Regulation höchst komplex ist und darüber hinaus vielschichtig mit diversen anderen Stoffwechselwegen in der Zelle vernetzt ist. Daher kann im Voraus keine Vorhersage gemacht werden, mit welcher Variante welcher Effekt auf das Wachstum eines Mikroorganismenstamms, die Balance seiner lebenswichtigen Stoffwechselabläufe und die Produktion von L-Methionin und SAM erzielt werden kann.

Aufgabe der vorliegenden Erfindung ist es, ein breites Spektrum neuer Varianten der Homoserin-Transsuccinylase (MetA-Protein) zur Verfügung zu stellen, die eine im Vergleich zum Wildtyp (WT) -Enzym erhöhte Feedback-Resistenz hinsichtlich L-Methionin und SAM besitzen.

Diese Aufgabe wird gelöst durch eine Homoserin-Transsuccinylase die im Vergleich zu einem Homoserin-Transsuccinylase Wildtyp-Enzym eine Mutation aufweist und eine im Vergleich zu dem Wildtyp-Enzym reduzierte Sensitivität gegenüber L-Methionin oder SAM zeigt, wobei das Wildtyp-Enzyms eine Aminosäuresequenz besitzt, die eine Teilsequenz AspGlyXaaXaaXaaThrGlyAlaPro zwischen Position 90 und 115 und eine Teilsequenz TyrGlnXaaThrPro zwischen Position 285 und 310 umfasst, wobei Position 1 der Aminosäuresequenz das Startmethionin ist, dadurch gekennzeichnet, dass die Mutation ein Aminosäureaustausch des Tyrosins in der Teilsequenz TyrGlnXaaThrPro ist.

Das Tyr in der Teilsequenz TyrGlnXaaThrPro ist bei einem Vergleich von Homoserin-Transsuccinylasen verschiedener Mikroorganismen konserviert. Der das konservierte Tyrosin Tyr enthaltende Sequenzbereich TyrGlnXaaThrPro liegt im MetA-Protein von E. coli zwischen Position 294 und 298 von SEQ ID No. 2. Er befindet sich in anderen MetA-Proteinen im Bereich zwischen Position 285 und 310. Xaa bedeutet eine beliebige natürliche Aminosäure.

Bisher ist die räumliche Struktur der Homoserin-Transsuccinylase nicht aufgeklärt. Daher ist die Zuordnung verschiedener Funktionen wie enzymatische Aktivität und Hemmbarkeit durch L-Methionin und/oder SAM zu bestimmten Aminosäuren nicht ermöglicht. Da die Faltung von Proteinen ein äußerst komplexer Vorgang ist, kann aus der Primärsequenz von Proteinen nicht auf eine räumliche Struktur geschlossen werden und es kommt nicht selten vor, dass Aminosäuren, die in der Primärsequenz weit voneinander entfernt sind, im gefalteten Protein in unmittelbarer Nähe liegen und umgekehrt. Überraschend wurde gefunden, dass der erfindungsgemäße Aminosäureaustausch an Position 294 des Proteins zu einer Herabsetzung der Feedback-Hemmbarkeit sowohl gegenüber L-Methionin als auch gegenüber SAM führt.

Eine erfindungsgemäße Homoserin-Transsuccinylase weist eine im Vergleich zum Wildtyp-Enzym verbesserte Resistenz (also erhöhter Ki) gegenüber den Inhibitoren SAM und/oder L-Methionin auf. Vorzugsweise weist sie eine im Vergleich zum Wildtyp zumindest 2fach erhöhte Resistenz gegenüber Methionin und/oder SAM auf. Besonders bevorzugt besitzt eine erfindungsgemäße Homoserin-Transsuccinylase eine im Vergleich zum Wildtyp 10fach erhöhte Resistenz, insbesondere bevorzugt eine 50fach erhöhte Resistenz gegenüber Methionin und/oder SAM, ganz besonders bevorzugt eine im Vergleich zu den in JP2000139471A genannten MetA-Mutanten erhöhte Resistenz.

Besonders bevorzugt umfasst die Proteinsequenz einer erfindungsgemäßen Homoserin-Transsuccinylase eine der in der Tabelle 1 aufgelisteten Mutationen oder eine Kombination der aufgeführten Mutationen.

Eine erfindungsgemäße Homoserin-Transsuccinylase kann beispielsweise durch Expression einer DNS-Sequenz, welche für eine erfindungsgemäße Homoserin-Transsuccinylase codiert, erhalten werden.

Die vorliegende Erfindung betrifft somit auch eine DNS-Sequenz, welche für eine erfindungsgemäße Homoserin-Transsuccinylase codiert.

Eine solche DNS-Sequenz ist erhältlich durch eine Mutation einer Base in einem oder mehreren Codonen eines metA-Gens, dadurch gekennzeichnet, dass im Codon für das konservierte Tyrosin, das sich im Wildtyp-Enzym von E. coli an Position 294 befindet, mindestens eine Mutation vorhanden ist, wobei Codon 1 mit der ersten Base aus Sequenz SEQ ID No. 1 beginnt.

Eine erfindungsgemäße DNS-Sequenz ist ein metA-Gen, bei dem das Codon für das konservierte Tyrosin Tyr in der Sequenz TyrGlnXaaThrPro, wobei diese Sequenz zwischen Position 285 und 310 liegt, verändert ist.

Im Folgenden wird eine erfindungsgemäße DNS-Sequenz als feedback-resistentes metA-Allel bezeichnet.

Im Rahmen der vorliegenden Erfindung sind als metA-Allele auch solche Gene aufzufassen, die bei einer Analyse mit dem Algorithmus BESTFIT (GCG Wisconsin Package, Genetics Computer Group (GLG) Madison, Wisconsin) eine Sequenzidentität von größer 50 % zum WT-metA-Gen von E. coli aufweisen. Ebenso sind Proteine mit einer Sequenzidentität von größer 50 % zur Wildtyp-Homoserin-Transsuccinylase von E. coli (Algorithmus BESTFIT, GCG Wisconsin Package, Genetics Computer Group (GLG) Madison, Wisconsin), die Homoserin-Transsuccinylase-Aktivität besitzen, als Homoserin-Transsuccinylasen aufzufassen.

Vorzugsweise umfasst ein erfindungsgemäßes metA-Allel eine in Tabelle 1, Spalte 2 bzw 4 aufgelistete Mutation oder eine Kombination der aufgeführten Mutationen.

Erfindungsgemäße metA-Allele lassen sich beispielsweise durch unspezifische oder durch gezielte Mutagenese aus im Folgenden beschriebenen Ausgangsmaterial herstellen. Unspezifische Mutationen innerhalb der genannten DNS-Region können zum Beispiel durch chemische Agentien (z. B. 1-Methyl-3-nitro-1-nitroso-guanidin, Ethylmethansulfonsäure u.ä.) und/oder durch physikalische Methoden und/oder durch unter bestimmten Bedingungen durchgeführte PCR-Reaktionen und/oder durch Amplifikation der DNS in Mutatorstämmen (z.B. XL1-Red) erzeugt werden. Methoden zur Einführung von Mutationen an spezifischen Positionen innerhalb eines DNS-Fragmentes sind bekannt. Eine weitere Möglichkeit zur Erzeugung feedback-resistenter metA-Allele besteht in der Kombination verschiedener, zur Feedback-Resistenz führender Mutationen zu multiplen Mutanten mit neuen Eigenschaften.

Als Ausgangsmaterial für die Mutagenese dient vorzugsweise die DNS eines Wildtyp-metA-Gens. Das zu mutierende metA-Gen kann chromosomal oder extrachromosomal codiert sein. Durch Anwendung der vorgenannten Mutagenese-Methoden werden ein oder mehrere Nukleotide der DNS-Sequenz so verändert, dass das nun durch das Gen codierte Protein eine Mutation des konservierten Tyrosins, das sich im Wildtyp-Enzym von E. coli an Position 294 befindet, aufweist, wobei Position 1 das Startmethionin aus SEQ ID NO. 2 ist.

Mit den beschriebenen Techniken lassen sich in ein beliebiges metA-Gen eine oder mehrere Mutationen im genannten DNS-Bereich einführen. Diese Mutationen bewirken, dass die codierte Homoserin-Transsuccinylase eine zur Feedback-Resistenz gegenüber SAM und/oder L-Methionin führende Aminosäuresequenz besitzt.

Im Anschluss an die beispielsweise wie beschrieben durchgeführte Mutagenese erfolgt die Selektion der Mutanten mit dem gewünschten Phänotyp beispielsweise durch Bestimmung des Ausmaßes der L-Methionin- und/oder SAM-Sensitivität der mutierten Homoserin-Transsuccinylasen.

Für die Bestimmung der L-Methionin- und/oder SAM-Sensitivität der Homoserin-Transsuccinylase kann jede Methode benützt werden, die es erlaubt, die Aktivität des Enzyms in Anwesenheit von L-Methionin oder SAM zu bestimmen. Beispielsweise kann die Bestimmung der Homoserin-Transsuccinylase-Aktivität in Anlehnung an die von Kredich und Tomkins beschriebene Methode zur Bestimmung der Aktivität von Serin-Acetyltransferasen (Kredich N.M. und Tomkins G.M., J. Biol. Chem. 241, 4955-4965 (1966)) erfolgen. Die Enzymaktivität wird in einem Ansatz, der Homoserin und Succinyl-CoA enthält, gemessen. Die Reaktion wird durch Enzymzugabe gestartet und über die Abnahme der Extinktion bei 232 nm, die durch Spaltung der Thioesterbindung im Succinyl-Coenzym A hervorgerufen wird, in einem Spektralphotometer verfolgt. Der beschriebene Test eignet sich für die Bestimmung der L-Methionin-Sensitivität der Homoserin-Transsuccinylasen. Die Hemmung der Homoserin-Transsuccinylase-Aktivität wird in Anwesenheit verschiedener Konzentrationen von L-Methionin im Reaktionsansatz getestet. Die katalytische Aktivität der verschiedenen Homoserin-Transsuccinylasen wird in An- und Abwesenheit von L-Methionin bestimmt und daraus die Hemmkonstante Ki ermittelt, welche diejenige Inhibitorkonzentration beschreibt, bei welcher die Aktivität nur noch 50 % der in Abwesenheit des Inhibitors messbaren beträgt.

Für die Bestimmung der SAM-Sensitivität der Aktivität der verschiedenen Homoserin-Transsuccinylasen kann beispielsweise ein wie in Lee L.W. et al., J. Biol. Chem. 241, 5479-5480 (1966) beschriebener Aktivitätstest erfolgen. Dabei wird der Enzymextrakt mit Homoserin und Succinyl-CoA inkubiert. Nach verschiedenen Zeitpunkten wird ein Teil des Testansatzes durch Zugabe zu einem Gemisch aus Ethanol, Wasser und 5,5'-Dithiobis(2-Nitrobenzoesäure) gestoppt. Die Absorption wird bei 412 nm photometrisch bestimmt. Der beschriebene Test eignet sich beispielsweise für die Bestimmung der SAM-Sensitivität der Homoserin-Transsuccinylasen. Die Hemmung der Homoserin-Transsuccinylase-Aktivität wird in Anwesenheit verschiedener Konzentrationen von SAM im Reaktionsansatz getestet. Die katalytische Aktivität der verschiedenen Homoserin-Transsuccinylasen wird in An- und Abwesenheit von SAM bestimmt und daraus die Hemmkonstante Ki ermittelt.

In der Regel bevorzugt wird eine Homoserin-Transsuccinylase mit einer verringerten L-Methionin- und/oder SAM-Sensitivität bei gleichzeitig unveränderter katalytischer Aktivität. Für andere Vorhaben kann eine gleichzeitige Reduzierung der L-Methionin- und/oder SAM-Sensitivität und der katalytischen Aktivität erstrebenswert sein.

Ein weiterer Gegenstand der Erfindung sind Mikroorganismenstämme, welche erfindungsgemäße feedback-resistente metA-Allele enthalten. Solche Stämme von Mikroorganismen sind dadurch gekennzeichnet, dass sie einen zumindest durch ein feedback-resistentes metA-Allel deregulierten L-Methionin- bzw. SAM-Stoffwechsel besitzen. Da bei allen Mikroorganismen dieser Stoffwechsel über denselben, an sich bekannten Weg verläuft und die zur Herstellung der erfindungsgemäßen Stämme anzuwendenden Techniken z. B. aus Standardlehrbüchern allgemein bekannt und auf alle Mikroorganismen anwendbar sind, sind erfindungsgemäße Stämme aus beliebigen Mikroorganismen herstellbar. Bevorzugt geeignet zur Herstellung eines erfindungsgemäßen Stammes sind Bakterien. Besonders bevorzugt geeignet sind gram-negative Bakterien, insbesondere E. coli.

Ein weiterer Gegenstand der Erfindung ist die Herstellung von L-Methionin oder SAM durch Kultivierung erfindungsgemäßer Mikroorganismen, außerdem die Verwendung erfindungsgemäßer Mikroorganismen zur Herstellung von Produkten, die Methionin enthalten (wie beispielsweise Methionin-enthaltende Peptide) oder sich im Stoffwechsel der Mikroorganismen von L-Methionin oder SAM ableiten (wie beispielsweise Polyamine, Liponsäure, Biotin und Chinone). Des weiteren können erfindungsgemäße Mikroorganismen, die SAM in im Vergleich zum Wildtyp verstärktem Maße produzieren, dazu verwendet werden, Produkte, die durch Übertragung der Methylgruppe von SAM entstehen, herzustellen.

Die feedback-resistenten metA-Allele werden zur Expression des veränderten Homoserin-Transsuccinylase-Enzyms mittels üblicher Verfahren in einen Wirtsstamm transformiert.

Die Expression eines feedback-resistenten metA-Allels kann unter Kontrolle des eigenen, vor dem metA-Gen lokalisierten Promotors oder durch Verwendung anderer geeigneter Promotorsysteme, die dem Fachmann bekannt sind, erfolgen. Dabei kann sich das entsprechende Gen unter der Kontrolle eines solchen Promotors entweder in einer oder in mehreren Kopien auf dem Chromosom des Wirtsorganismus oder auf einem Vektor, vorzugsweise einem Plasmid befinden. Die Erfindung betrifft daher auch ein Plasmid, dadurch gekennzeichnet, dass es ein erfindungsgemäßes feedback-resistentes metA-Allel mit einem Promotor enthält.

Zur Klonierung können Vektoren verwendet werden, die bereits genetische Elemente (z.B. konstitutive oder regulierbare Promotoren, Terminatoren) enthalten, die entweder eine andauernde oder eine kontrollierte, induzierbare Expression des für eine Homoserin-Transsuccinylase codierenden Gens ermöglichen. Außerdem befinden sich auf einem Expressionsvektor vorzugsweise andere regulatorische Elemente wie ribosomale Bindungsstellen und Terminationssequenzen sowie Sequenzen, die für selektive Marker und/oder Reporter-Gene codieren. Die Expression derartiger Selektionsmarker erleichtert die Identifizierung von Transformanten. Als Selektionsmarker geeignet sind Gene, die für eine Resistenz gegenüber z. B. Ampicillin, Tetracyclin, Chloramphenicol, Kanamycin oder andere Antibiotika codieren. Wenn das erfindungsgemäße metA-Allel extrachromosomal repliziert werden soll, sollte der Plasmidvektor vorzugsweise einen Ursprungspunkt der Replikation enthalten. Besonders bevorzugt sind Plasmid-Vektoren wie beispielsweise die E. coli-Vektoren pACYC184, pUC18, pBR322, pSC101 und ihre Derivate. Als induzierbare Promotoren eignen sich beispielsweise der lac-, tac-, trc-, lambda PL, ara- oder tet-Promotor oder davon abgeleitete Sequenzen. Bevorzugt wird die konstitutive Expression von einem GAPDH-Promotor. In einer besonders bevorzugten Ausführungsform der vorliegenden Erfindung befinden sich die für die Homoserin-Transsuccinylase codierenden Gene unter Kontrolle des GAPDH-Promoters in einem von pACYC184 abgeleiteten Plasmid. Die Strategien zur Integration von Genen in das Chromosom sind Stand der Technik.

Ein geeigneter Wirtsstamm wird mit einem Expressionsvektor, der die für eine L-Methionin- und/oder SAM-insensitive Homoserin-Transsuccinylase codierende Transkriptionseinheit enthält, transformiert. Als Wirtsstämme werden Stämme, die L-Methionin- und/oder SAM-sensitive Proteine enthalten, wie zum Beispiel Bakterien verwendet.

Als Wirtsstamm wird vorzugsweise ein E. coli-Wildtypstamm oder ein Stamm verwendet, in dem das endogene metA-Gen inaktiviert ist, wie z.B. E. coli Stamm DL41, CGSC-Stammsammlung Nr. 7177. Solche Stämme werden durch ein erfindungsgemäßes metA-Gen komplementiert. Die Fähigkeit eines erfindungsgemäßen Stammes zur mikrobiellen Produktion von L-Methionin oder SAM kann durch zusätzliche Maßnahmen verstärkt werden. Beispielsweise können zu diesem Zweck Stämme verwendet werden, in welchen das Gen metJ, welches für einen Repressor der Gene des Methionin-Stoffwechsels codiert, nicht mehr exprimiert wird (JP2000139471A).

Die Produktion von L-Methionin oder SAM erfolgt vorzugsweise durch Kultivierung eines erfindungsgemäßen Mikroorganismenstammes. Dazu wird der Mikroorganismenstamm beispielsweise in einem Fermenter in einem Nährmedium kultiviert, das eine geeignete Kohlenstoff-, und eine geeignete Energiequelle, sowie andere Zusatzstoffe enthält.

Die während der Fermentation gebildeten Substanzen wie beispielweise L-Methionin oder SAM können anschließend aufgereinigt werden.

Die folgenden Beispiele dienen der weiteren Erläuterung der Erfindung. Sämtliche eingesetzten molekularbiologischen Verfahren, wie Polymerase-Kettenreaktion, Isolierung und Reinigung von DNS, Modifikation von DNS durch Restriktionsenzyme, Klenow-Fragment und Ligase, Transformation etc wurden in der dem Fachmann bekannten, in der Literatur beschriebenen oder von den jeweiligen Herstellern empfohlenen Art und Weise durchgeführt.

### Beispiel 1:

### Erzeugung von feedback-resistenten Homoserin-Transsuccinylasen durch ungerichtete Mutagenese des metA-Strukturgens

Das Gen metA aus E. coli wurde durch Polymerase-Kettenreaktion unter Verwendung der am 5'-Ende phosphorylierten Oligonukleotide metAfw mit der Sequenz
5'-GATCCCATGGCTCCTTTTAGTCATTCTTAT-3', (SEQ ID No. 3)
und metArev mit der Sequenz
5'-GATCGAGCTCAGTACTATTAATCCAGCGTTGGATTC-3', (SEQ ID No. 4)
als Primer und chromosomaler DNS aus E. coli Stamm W3110 (ATCC 27325) als Substrat amplifiziert. Das 1,1 kb lange Produkt wurde elektrophoretisch isoliert und mittels eines QIAquick Gel Extraction Kits (Qiagen) gereinigt. Danach wurde es in das Plasmid pBR322 (MBI Fermentas), das mit dem Restriktionsenzym EcoRI und dem Klenow-Fragment (Roche) behandelt worden war, mittels T4-DNS-Ligase eingefügt. Das entstandene Plasmid pKP438 wurde für die Mutagenese eingesetzt.

Plasmid pKP438 wurde durch Transformation in den E. coli-Stamm XL1-Red (Stratagene) eingebracht und durch Kultivierung nach Anleitung des Herstellers wurden Mutationen im Plasmid pKP438 eingeführt. Die Mutagenese erfolgte in Gegenwart von kritischen Konzentrationen von Methionin-Analoga wie in Lawrence D.A. und Smith D.A., Genetics 58: 473-492 (1968) beschrieben. Durch diese Prozedur werden Mutanten selektiert, die eine Methionin-Überproduktion zeigen. Die meisten dieser Mutanten sind auf veränderte, auf dem Plasmid pKP438 codierte Homoserin-Transsuccinylasen zurückzuführen.

Das Plasmid aus einer Mutante wurden isoliert und die DNS-Sequenz des metA-Gens wurde bestimmt. Es zeigte sich, dass das Gen im Vergleich zum Wildtyp einen Basenaustausch aufweist, der zu einer veränderten Aminosäure in der jeweils codierten Homoserin-Transsuccinylase führt. pBR3 enthält als Base 881 ein G statt dem im Wildtyp-Gen vorkommenden A, wodurch im codierten Protein an Position 294 Cystein statt Tyrosin eingebaut wird. Beispiel 2:

### Erzeugung von feedback-resistenten Homoserin-Transsuccinylasen durch gezielte Basenaustausche im metA-Strukturgen

In Beispiel 1 wurden metA-Allele hergestellt, die aufgrund von Basenaustausch und damit einhergehender Aminosäureveränderung an Position 294 gegenüber L-Methionin und/oder SAM feedback-resistente Homoserin-Transsuccinylasen codieren (siehe Beispiele 3 und 4). Durch ortsspezifische Mutagenese wurden daher Gene konstruiert, die für Homoserin-Transsuccinylasen codieren, in denen die Aminosäure Tyrosin an Position 294 durch verschiedene andere Aminosäuren ersetzt ist und die dadurch veränderte Eigenschaften hinsichtlich der Hemmung ihrer Aktivität durch L-Methionin und SAM aufweisen.

Als Basisplasmid für die Konstruktion der erfindungsgemäßen Plasmide wurde das von pACYC184 abgeleitete Plasmid pACYC184-LH verwendet, welches unter der Nummer DSM 10172 bei der Deutschen Sammlung für Mikroorganismen und Zellkulturen in Braunschweig hinterlegt ist. In dieses Plasmid wurde die Sequenz des GAPDH-Promotors und zusätzlich davor eine NdeI-Schnittstelle durch folgendes Vorgehen eingefügt: Der GAPDH-Promotor wurde durch Polymerase-Kettenreaktion nach den dem Fachmann bekannten Regeln amplifiziert, wobei die Oligonukleotide GAPDHfw mit der Sequenz
5'-GTCGACGCGTGAGGCGAGTCAGTCGCGTAATGC-3' (SEQ ID No. 5) und GAPDHrevII mit der Sequenz
5'-GACCTTAATTAAGATCTCATATGTTCCACCAGCTATTTGTTA-3' (SEQ ID No. 6)
als Primer und chromosomale DNS aus E. coli Stamm W3110 (ATCC 27325) als Substrat dienten. Das Produkt wurde elektrophoretisch isoliert, mittels eines QIAquick Gel Extraction Kits (Qiagen) gereinigt und mit den Restriktionsenzymen MluI und PacI nach Herstellerangaben behandelt. Hierauf wurde es in einen mit den gleichen Enzymen behandelten Vektor pACYC184-LH mit Hilfe von T4-Ligase eingefügt, wodurch das Plasmid pKP290 entstand.

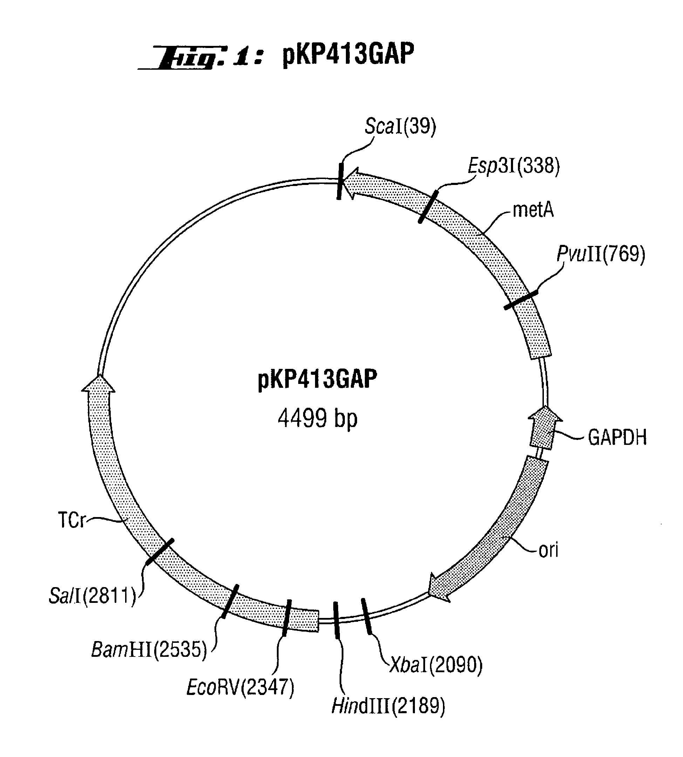

Das Gen metA aus E. coli wird durch Polymerase-Kettenreaktion unter Verwendung der Oligonukleotide metAfw2 mit der Sequenz 5'-CATGGCTCCTTTTAGTCATTCTTATATTCTAACGTAG-3', (SEQ ID No. 7) und metArev2 mit der Sequenz
5'-ACGCGTATGCATCCAGAGCTCAGTACTATTAATCCAGCGTTGGATTC-3', (SEQ ID No. 8)
als Primer und chromosomaler DNS aus E. coli Stamm W3110 (ATCC 27325) als Substrat amplifiziert. Das 1,1 kb lange Produkt wurde elektrophoretisch aufgetrennt und mittels QIAquick Gel Extraction Kit (Qiagen) gereinigt. Danach wurde das Produkt in den folgendermaßen vorbereiteten Vektor pKP290 ligiert: Behandlung mit Restriktionsenzym NdeI, Klenow-Enzym, Restriktionsenzym BglII und Mung Bean Nuclease (Roche). Das entstandene Plasmid pKP413GAP ist in Fig. 1 dargestellt und unter der Nummer DSM 15221 bei der Deutschen Sammlung für Mikroorganismen und Zellkulturen in Braunschweig hinterlegt. Es enthält das metA-Gen aus E. coli unter Kontrolle des GAPDH-Promotors und dient als Ausgangsplasmid zur Herstellung der feedback-resistenten metA-Allele.

Plasmid pKP413GAP wurde einer gerichteten ortsspezifischen Mutagenese betreffend Codon 294 des metA-Strukturgens unterzogen. Dazu wurde eine inverse Polymerase-Kettenreaktion mit Pfu-Polymerase (Promega) nach dem Fachmann bekannten Regeln durchgeführt. Als Primer dienten die am 5'-Ende phosphorylierten Oligonukleotide metAmutfw1 mit der Sequenz
5'-NNNCAGATCACGCCATACGATCTAC-3' (SEQ ID No. 9), wobei bei der Synthese für N eine 1:1:1:1 Mischung aus A,T,C,G eingesetzt wurde und metAmutrev1 mit der Sequenz
5'-GACGTAATAGTTGAGCCAGTTGG-3' (SEQ ID No. 10). Das etwa 4,3 kb große Produkt wurde elektrophoretisch isoliert und mittels eines QIAquick Gel Extraction Kits (Qiagen) nach Herstellerangaben gereinigt. Danach erfolgte eine intramolekulare Ligation mit T4-DNS-Ligase nach Herstellerangaben. Die Transformation von E. coli-Zellen des Stammes DH5α erfolgte nach der CaCl₂-Methode auf dem Fachmann bekannte Art und Weise. Der Transformationsansatz wurde auf LB-Tetracyclin-Agarplatten (10 g/l Trypton, 5 g/l Hefeextrakt, 10 g/l NaCl, 15 g/l Agar, 15 mg/l Tetracyclin) ausgebracht und über Nacht bei 37 °C inkubiert. Die gewünschten Transformanten wurden nach einer Plasmidisolierung mittels eines QIAprep Spin Miniprep Kit (Qiagen) durch eine Restriktionsanalyse identifiziert. Der Bereich zwischen den Schnittstellen Esp3I und ScaI, der das Codon 294 des metA-Strukturgens enthält, wurde sequenziert, isoliert und in ein mit den gleichen Enzymen behandeltes Plasmid pKP413GAP mittels dem Fachmann bekannten Methoden eingefügt.

Die so konstruierten Plasmide enthalten das vollständige metA-Gen mit jeweils im Vergleich zur Wildtyp-Sequenz veränderter Sequenz an Codon 294, wodurch sie für die Produktion verschiedener Varianten von Homoserin-Transsuccinylasen eingesetzt werden können (Tabelle 1).

**Tabelle 1: Ausgangsplasmid sowie Plasmide enthaltend metA-Varianten mit verändertem Codon 294.**

| Plasmid | Codon 101 | Aminosäure 101 | Codon 294 | Aminosäure 294 |
|---|---|---|---|---|
| | | | | |
| pKP413GAP | GAC | Asp | TAC | Tyr |
| | | | | |
| pKP446 | GAC | Asp | TGC | Cys |
| pSLmetA*L | GAC | Asp | CTC | Leu |
| pSLmetA*A | GAC | Asp | GCC | Ala |
| pSLmetA*P | GAC | Asp | CCT | Pro |
| pSLmetA*Q | GAC | Asp | CAG | Gln |
| pSLmetA*K | GAC | Asp | AAG | Lys |
| pSLmetA*O | GAC | Asp | ---*) | -**) |

| | | | | |
|---|---|---|---|---|
| *): Codon 294 fehlt in metA-Sequenz **): die Aminosäure 294 ist deletiert | | | | |

### Beispiel 3:

### Aktivität der Homoserin-Transsuccinylase-Mutanten und Feedback-Resistenz gegenüber L-Methionin

Die Aktivität und der Einfluss von L-Methionin auf die Aktivität der verschiedenen Homoserin-Transsuccinylasen wurde durch einen Enzymtest mit Zellextrakten, in denen die jeweiligen Proteine produziert worden waren, bestimmt. Dazu wurden die entsprechenden für veränderte Homoserin-Transsuccinylasen codierenden Plasmide mittels Transformation nach dem Fachmann bekannten Methoden in den E. coli-Stamm W3110 (ATCC 27325) eingebracht. Der Transformationsansatz wurde auf LB-Tetracyclin-Agarplatten (10 g/l Trypton, 5 g/l Hefeextrakt, 5 g/l NaCl, 15 g/l Agar, 15 mg/l Tetracyclin) ausgebracht und über Nacht bei 37 °C inkubiert. Die erhaltenen Transformanten wurden in SM1-Medium (für 1 l Medium: CaCl₂ x 2 H₂O 0,0147 g, MgSO₄ x 7 H₂O 0,3 g, Na₂MoO₄ x 2 H₂O 0,15 mg, H₃BO₃ 2,5 mg, CoCl₂ x 6 H₂O 0,7 mg, CuSO₄ x 5 H₂O 0,25 mg, MnCl₂ x 4 H₂O 1,6 mg, ZnSO₄ x 7 H₂O 0,3 mg, KH₂PO₄ 3, 0 g, K₂HPO₄ 12,0 g, (NH₄)₂SO₄ 5 g, NaCl 0,6 g, FeSO₄ x 7 H₂O 0,002 g, Na₃-Citrat x 2 H₂O 1g, Glucose 5 g, Trypton 1 g, Hefeextrakt 0,5 g) angezogen, bei einer Absorption von ca. 0,8 bei 600 nm abzentrifugiert, in 50 mM Tris pH 7,5 gewaschen und erneut abzentrifugiert. Die Zellen wurden in 50 mM Tris/Cl pH 7,5, 2 mM Dithiothreitol, 0,5 mM Phenyl-Methyl-Sulfonsäurefluorid resuspendiert und in einer French Press aufgebrochen. Der Überstand einer weiteren Zentrifugation wurde als Enzymextrakt in den Test eingesetzt. Die Enzymaktivität wurde in einem Ansatz mit 50 mM Tris/Cl pH 7,6, 1 mM Homoserin und 0,1 mM Succinyl-CoA bestimmt, indem das bei der Reaktion entstehende Coenzym A über die Abnahme der Extinktion bei 232 nm photometrisch quantifiziert wurde in Anlehnung an die von Kredich und Tomkins beschriebene Methode zur Bestimmung der Aktivität von Serin-Acetyltransferasen, (Kredich N.M. und Tomkins G.M., J. Biol. Chem. 241, 4955-4965 (1966)). Die Auswirkung von zugesetztem L-Methionin auf die Aktivität wurde bestimmt und die Hemmbarkeit wurde als Ki quantifiziert. Als Ki wird diejenige Konzentration an L-Methionin bestimmt, bei der die Aktivität der Homoserin-Transsuccinylase nur noch 50% der Aktivität in Abwesenheit von L-Methionin beträgt.
Alle Homoserin-Transsuccinylase-Mutanten zeigen eine im Vergleich zum Wildtyp erhöhte Feedback-Resistenz hinsichtlich L-Methionin. Tabelle 2 zeigt eine Zusammenfassung der Ergebnisse.

**Tabelle 2: Aktivität des WT-Enzyms sowie der Homoserin-Transsuccinylase-Mutanten und Feedback-Resistenz gegenüber L-Methionin.**

| Plasmid | Aktivität (U/mg) | Aktivität (%)* in Anwesenheit von 1 mM L-Methionin | Ki L-Methionin (mM) |
|---|---|---|---|
| | | | |
| pKP413GAP | 0,155 | 2 | 0,05 |
| | | | |
| pKP446 | 0,133 | 96 | 11 |
| pSLmetA*L | 0,070 | 89 | 6,5 |
| pSLmetA*A | 0,063 | 94 | 7,5 |
| pSLmetA*P | 0,020 | 91 | 6 |
| pSLmetA*Q | 0,065 | 95 | 11 |
| pSLmetA*K | 0,048 | 92 | 12,5 |
| pSLmetA*O | 0,085 | 98 | 14,5 |

| | | | |
|---|---|---|---|
| * Aktivität in Abwesenheit von L-Methionin entspricht 100%. | | | |

### Beispiel 4:

### Feedback-Resistenz der Homoserin-Transsuccinylasen gegenüber SAM

Der Einfluß von SAM auf die Aktivität der verschiedenen Homoserin-Transsuccinylasen wurde durch Quantifizierung der Aktivität in Gegenwart verschiedener SAM-Konzentrationen (Cl-Salz, Sigma) bestimmt. Die Anzucht und Präparation der Zellextrakte erfolgte wie in Beispiel 3 beschrieben. Der Aktivitätstest erfolgte wie in Lee L.W. et al., J. Biol. Chem. 241, 5479-5480 (1966) beschrieben, wobei der Enzymextrakt mit 50 mM Kaliumphosphat-Puffer pH 7,5, 3 mM Homoserin und 0,3 mM Succinyl-CoA inkubiert wurde. Nach verschiedenen Zeitpunkten wurden 100 µl Testansatz durch Zugabe zu einem Gemisch aus 400 µl Ethanol, 400 µl Wasser und 100 µl 10 mM 5,5'-Dithiobis(2-Nitrobenzoesäure) gestoppt. Nachdem der Ansatz 5 Minuten bei Raumtemperatur inkubiert wurde, wurde die Absorption bei 412 nm photometrisch bestimmt. Nach Bestimmung der Proteinkonzentration wurde unter Verwendung des Extinktionskoeffizienten die Enzymaktivität errechnet. Als Maß für die Hemmbarkeit der Aktivität durch SAM wurde der Ki bestimmt.

**Tabelle 3: Aktivität der Homoserin-Transsuccinylase-Mutanten und Feedback-Resistenz gegenüber SAM.**

| Plasmid | Aktivität (U/mg) | Aktivität (%)* in Gegenwart von 1 mM SAM | Ki SAM (mM) |
|---|---|---|---|
| | | | |
| pKP413GAP | 0,62 | 0,5 | 0,2 |
| | | | |
| pKP446 | 0,49 | 92 | 10 |
| pSLmetA*L | 0,29 | 80 | 7 |
| pSLmetA*A | 0,26 | 95 | 10 |
| pSLmetA*P | 0,15 | 98 | 18 |
| pSLmetA*Q | 0,21 | 87 | 6 |
| pSLmetA*K | 0,14 | 90 | 5 |
| pSLmetA*O | 0,33 | 96 | 10 |

| | | | |
|---|---|---|---|
| * Aktivität in Abwesenheit von SAM entspricht 100%. | | | |

## Patentansprüche

1. Homoserin-Transsuccinylase, die im Vergleich zu einem Homoserin-Transsuccinylase-Wildtyp-Enzym eine Mutation aufweist und eine im Vergleich zu dem Wildtyp-Enzym reduzierte Sensitivität gegenüber L-Methionin oder SAM zeigt, wobei das Wildtyp-Enzyms eine Aminosäuresequenz besitzt, die eine Teilsequenz AspGlyXaaXaaXaaThrGlyAlaPro zwischen Position 90 und 115 und eine Teilsequenz TyrGlnXaaThrPro zwischen Position 285 und 310 umfasst, wobei Position 1 der Aminosäuresequenz das Startmethionin ist, **dadurch gekennzeichnet, dass** die Mutation ein Aminosäureaustausch des Tyrosins in der Teilsequenz TyrGlnXaaThrPro ist.

2. Homoserin-Transsuccinylase nach Anspruch 1, **dadurch gekennzeichnet, dass** sie eine im Vergleich zum Wildtyp zumindest 2-fach erhöhte Resistenz (erhöhter Ki) gegenüber SAM oder L-Methionin aufweist.

3. Homoserin-Transsuccinylase nach Anspruch 1 oder 2, **dadurch gekennzeichnet, dass** sie eine der in Tabelle 1 aufgelisteten Mutationen enthält.

4. MetA-Allel codierend für eine Homoserin-Transsuccinylase gemäß einem der Ansprüche 1 bis 3.

5. Plasmid, **dadurch gekennzeichnet, dass** es ein metA-Allel gemäß Anspruch 4 mit einem Promotor enthält.

6. Mikroorganismenstamm, **dadurch gekennzeichnet, dass** er ein feedback-resistentes metA-Allel gemäß Anspruch 4 enthält.

7. Mikroorganismenstamm, gemäß Anspruch 6 **dadurch gekennzeichnet, dass** es sich um einen gram-negativen Bakterienstamm, vorzugsweise um E. coli handelt.

8. Verfahren zur Herstellung von L-Methionin oder SAM durch Kultivierung eines Mikroorganismenstammes gemäß Anspruch 6 oder 7.

## Claims

1. Homoserine transsuccinylase which possesses a mutation as compared with a homoserine transsuccinylase wild-type enzyme and exhibits a reduced sensitivity toward L-methionine or SAM as compared with the wild-type enzyme, with the wild-type enzyme possessing an amino acid sequence which comprises a constituent sequence AspGlyXaaXaaXaaThrGlyAlaPro between positions 90 and 115 and a constituent sequence TyrGlnXaaThrPro between positions 285 and 310, with position 1 of the amino acid sequence being the starting methionine, **characterized in that** the mutation is an amino acid replacement of the tyrosine in the constituent sequence TyrGlnXaaThrPro.

2. Homoserine transsuccinylase according to Claim 1, **characterized in that** it exhibits a resistance toward SAM or L-methionine which is increased (increased Ki) at least 2-fold as compared with that of the wild type.

3. Homoserine transsuccinylase according to Claim 1 or 2, **characterized in that** it contains one of the mutations listed in Table 1.

4. MetA allele which encodes a homoserine transsuccinylase according to one of Claims 1 to 3.

5. Plasmid, **characterized in that** it contains a metA allele according to Claim 4 together with a promoter.

6. Microorganism strain, **characterized in that** it contains a feedback-resistant metA allele according to Claim 4.

7. Microorganism strain according to Claim 6, **characterized in that** it is a Gram-negative bacterial strain, preferably E. coli.

8. Method for preparing L-methionine or SAM by culturing a microorganism strain according to Claim 6 or 7.

## Revendications

1. Homosérine transsuccinylase qui présente, en comparaison d'une enzyme homosérine transsuccinylase de type sauvage, une mutation et, en comparaison de l'enzyme de type sauvage, une sensibilité réduite vis-à-vis de la L-méthionine ou de la SAM (S-adénosylméthionine), l'enzyme de type sauvage possédant une séquence d'acides aminés qui englobe une séquence partielle de type AspGlyXaaXaaXaaThrGlyAlaPro entre la position 90 et la position 115 et une séquence partielle de type TyrGlnXaaThrPro entre la position 285 et la position 310, où la position 1 de la séquence d'acides aminés est la méthionine de départ, **caractérisée en ce que** la mutation est un échange d'acides aminés de la tyrosine dans la séquence partielle TyrGlnXaaThrPro.

2. Homosérine transsuccinylase selon la revendication 1, **caractérisée en ce qu'**elle présente, en comparaison du type sauvage (Kᵢ supérieur), une résistance deux fois plus grande vis-à-vis de la SAM ou de la L-méthionine.

3. Homosérine transsuccinylase selon la revendication 1 ou 2, **caractérisée en ce qu'**elle contient l'une des mutations qui figurent dans le tableau I.

4. Allèle MetA codant pour une homosérine transsuccinylase selon l'une quelconque des revendications 1 à 3.

5. Plasmide, **caractérisé en ce qu'**il contient un allèle metA selon la revendication 4 avec un promoteur.

6. Souche de microorganismes, **caractérisée en ce qu'**elle contient un allèle metA selon la revendication 4 résistant à une rétroaction.

7. Souche de microorganismes selon la revendication 6, **caractérisée en ce qu'**il s'agit d'une souche de bactéries à gram négatif, de préférence, de E. coli.

8. Procédé de fabrication de L-méthionine ou de SAM en effectuant la culture d'une souche de microorganismes selon la revendication 6 ou 7.
